# EUROPEAN PATENT SPECIFICATION

(11) **EP 2 598 071 B1**
(45) Date of publication and mention of the grant of the patent: **14.10.2015**
(21) Application number: 11740795.7
(22) Date of filing: 29.07.2011
(51) Int. Cl.: A61B 18/18

(54) **RENAL NERVE ABLATION USING MILD FREEZING AND MICROWAVE ENERGY**
NIERENNERVENABLATION MIT LEICHTER GEFRIERUNG UND MIKROWELLENENERGIE
ABLATION DE NERF RÉNAL À L'AIDE DE CONGÉLATION LÉGÈRE ET D'ÉNERGIE MICRO-ONDE

(30) Priority: 28.07.2011 US 201113193437; 30.07.2010 US 369460 P
(43) Date of publication of application: 05.06.2013
(73) Proprietor: Boston Scientific Scimed, Inc., Maple Grove, MN 55311 (US)
(72) Inventor: SMITH, Scott, Chaska MN 55318 (US)
(74) Representative: Vossius & Partner Patentanwälte Rechtsanwälte mbB
(86) International application number: PCT/US2011/045881
(87) International publication number: WO 2012/016137

(56) References cited:
- WO-A2-2011/130534
- US-A1- 2009 076 409
- US-B1- 7 097 641

## Description

A system for thermally-induced renal neuromodulation employing cooling of non-targeted tissue is disclosed in US2009/0076409 A1.

### SUMMARY

Embodiments of the disclosure are directed to ablating target tissue of the body, such as innervated renal tissue or diseased tissue of an organ. Embodiments of the disclosure are directed to systems and apparatuses for ablating target tissue of the body, such as innervated renal tissue, using thermal and microwave elements of an intravascular catheter arrangement. Embodiments of the disclosure are directed to systems and apparatuses for ablating innervated renal tissue using thermal and microwave elements of an intravascular catheter configured to cause formation of ice particles within the renal artery wall and ablate perivascular nerve tissue using microwave energy radiation without injuring the renal artery wall.

Embodiments are directed to an apparatus comprising a catheter arrangement which includes a flexible shaft and a cooling arrangement provided at a distal end of the shaft. The cooling arrangement is configured to freeze non-targeted body tissue while neighboring target body tissue remains unfrozen. A microwave emitter is configured to propagate microwave energy through the frozen non-targeted tissue and to heat the unfrozen target tissue to a temperature sufficient to ablate the target tissue with no or negligible thermal damage to the non-targeted tissue. In various embodiments, the catheter arrangement includes a balloon within which at least the cooling arrangement is situated. In other embodiments, the catheter arrangement includes a balloon within which the cooling arrangement and the microwave emitter are situated.

An external system can be coupled to the proximal end of the catheter arrangement, and configured to control power delivered to the microwave emitter and coolant delivered to the cooling arrangement. A device can be used to verify that the non-targeted tissue is frozen and that the target tissue is unfrozen. Suitable devices include a temperature probe which may include a tissue penetrating member, and an ultrasound device configured to measure a depth of ice formation within the non-targeted tissue.

According to various embodiments, an apparatus includes a catheter arrangement comprising a flexible shaft having a proximal end, a distal end, a length, and a lumen arrangement extending between the proximal and distal ends. The length of the shaft is sufficient to access a patient's renal artery relative to a percutaneous access location. A cooling arrangement is provided at a distal end of the shaft, coupled to the lumen arrangement, and dimensioned for deployment within a renal artery. The cooling arrangement is configured to freeze tissue of a wall of the renal artery while target tissue adjacent the renal artery wall including perivascular renal nerve tissue remains unfrozen. A microwave emitter is configured to propagate microwave energy through the frozen renal artery wall to heat the unfrozen target tissue to a temperature sufficient to ablate perivascular renal nerve tissue included within the target tissue with no or negligible thermal damage to at least an inner wall of the renal artery.

In some embodiments, the microwave emitter includes a transmission antenna configured to radiate microwave energy through the frozen renal artery wall and into the unfrozen target tissue. The cooling arrangement may be configured to receive a cryogen fluid via the lumen arrangement, comprise a phase-change cryothermal apparatus configured to receive a cryogen liquid and output spent gas resulting from the cryothermal phase-change, comprise a heat exchange apparatus configured to receive a liquid coolant capable of causing freezing of the renal artery wall, or comprise a solid-state thermoelectric cooling device, for example.

Various examples are directed to methods involving microwave ablation of target tissue of the body. According to various examples, an ablation method involves applying cooling to non-targeted tissue of the body to achieve a tissue temperature below freezing while target tissue adjacent the non-targeted tissue remains unfrozen. The ablation method further involves propagating microwave energy through the frozen non-targeted tissue to heat the unfrozen target tissue to a temperature sufficient to ablate at least some of the target tissue with no or negligible thermal damage to the non-targeted tissue. In some embodiments, the non-targeted tissue comprises tissue of a wall of a vessel in the body, and the target tissue comprises body tissue adjacent the vessel wall.

Preferably, an ablation method involves applying cooling to a wall of a patient's renal artery to lower the artery wall temperature to below freezing while target tissue adjacent the renal artery wall including perivascular renal nerve tissue remains unfrozen. The ablation method further involves propagating microwave energy through the frozen renal artery wall to heat the unfrozen target tissue to a temperature sufficient to ablate perivascular renal nerve tissue included within the target tissue with no or negligible thermal damage to at least an inner wall of the renal artery. The ablation method may further involve verifying that the renal artery wall is frozen and that the target tissue is unfrozen.

These and other features can be understood in view of the following detailed discussion and the accompanying drawings.

### BRIEF DESCRIPTION OF THE DRAWINGS

Figure 1 is an illustration of a right kidney and renal vasculature including a renal artery branching laterally from the abdominal aorta;
Figures 2A and 2B illustrate sympathetic innervation of the renal artery;
Figure 3A illustrates various tissue layers of the wall of the renal artery;
Figures 3B and 3C illustrate a portion of a renal nerve;
Figure 4A shows a therapy device of an ablation catheter which includes a thermal unit and a microwave emitter in accordance with various embodiments;
Figure 4B shows a therapy device of an ablation catheter which includes a thermal unit, a microwave emitter, and an ultrasound device in accordance with various embodiments;
Figures 4C and 4D show different ultrasound device configurations in accordance with various embodiments;
Figure 5 shows a therapy unit which incorporates a cooling arrangement, a microwave emitter, and one or more aperture sensors in accordance with various embodiments;
Figure 6A shows a microwave emitter which is configured to radiate a circumferential electromagnetic field in accordance with various embodiments;
Figure 6B shows a microwave emitter which is configured to radiate an electromagnetic field having a prescribed radiation pattern in accordance with various embodiments;
Figure 6C shows a therapy device deployed in a renal artery having a microwave emitter configured to radiate a circumferential electromagnetic field in accordance with various embodiments;
Figure 7 shows a lumen arrangement within a flexible shaft of a therapy catheter in accordance with various embodiments; and
Figure 8 shows a therapy system configured to perform renal denervation using combined cooling and microwave emission in accordance with various embodiments.

### DESCRIPTION

Embodiments of the disclosure are directed to apparatuses that protect non-target tissue while ablating neighboring target tissue. Embodiments are directed to apparatuses that protectively freeze non-target tissue while ablating neighboring unfrozen target tissue. Embodiments are directed to apparatuses that protectively freeze non-target tissue while ablating neighboring unfrozen target tissue using microwave energy radiation. Embodiments are directed to apparatuses and methods for ablating perivascular renal nerves for treatment of hypertension using microwave energy radiation while thermally protecting renal artery wall tissue using mild freezing, and for ablation of other body tissues, such as diseased prostate tissue (e.g., prostate ablation for benign prostatic hypertrophy) and cardiac chamber and vessel tissue (e.g., atrial ablation for treating atrial fibrillation).

It has been difficult to effectively ablate perivascular renal sympathetic nerves by access from the renal artery, without injury to the renal artery wall. To reduce concern for potential stenotic narrowing of the artery after the ablation procedure, minimizing arterial injury during such an ablation procedure is important. Because some of the renal nerves can be located immediately adjacent the artery, or even in the arterial adventitia, there is need for a sharp transition between the protected tissue and the ablated tissue to allow more effective nerve ablation with minimized arterial wall injury.

Embodiments of the disclosure exploit the property that microwaves heat liquid water molecules much more so than ice, and this property can be used to direct the energy distribution and absorption by tissues when using an intravascular microwave ablation catheter. A mild freezing of the artery wall allows microwave energy to pass through the artery wall to preferentially heat the non-frozen adjacent tissues including renal sympathetic nerves. The nerves can be effectively treated without significant heating of tissue of the artery wall. The therapy catheter can be utilized for wall protection during adjacent tissue ablation with a microwave catheter situated in the renal vein or ureter, individually or in combination.

For example, external microwave energy can be focused to heat the target region including the renal artery and nearby nerves, with an intravascular catheter placed to freeze a narrow layer of the renal artery including the artery wall, thereby protecting the artery from thermal injury resulting from the microwave tissue heating. This approach can be used to protect other tissues in contact with a catheter while ablating tissues farther out from the catheter, such as for prostate ablation for BPH (benign prostatic hypertrophy), tumor ablation, or other hyperthermia or hypothermia therapies.

According to various embodiments, a therapy catheter is configured for placement in the renal artery. The therapy catheter includes an emitter (e.g., an antenna) configured to transmit microwave energy radiation to target tissue, with cryothermal cooling provided to maintain intervening tissue in a mildly frozen state. A phase-change cryothermal mechanism can be used, or a simpler heat exchanger system with liquid coolant can be used. Cryothermal cooling may also be achieved using solid-state cooling devices, such as thermoelectric cooling devices.

In some embodiments, a therapy catheter incorporates a phase-change cryothermal capability such as by spraying a cryogen to cool an inflated balloon. The therapy catheter also includes a microwave transmission antenna apparatus for delivering microwave energy locally to the region near the active portion of the catheter. Temperature and pressure sensors or other sensor elements (e.g., impedance sensors) can be incorporated to facilitate monitoring and control of the ablation procedure. The cryothermal and microwave transmission components of the therapy catheter interface with external control units to control device functioning and monitor or display temperatures, power used, blood pressure, or other parameters.

In other embodiments, the therapy catheter incorporates a heat exchange apparatus configured to receive a liquid coolant capable of causing freezing of tissue proximate of the target tissue, such as the wall of the renal artery. In some embodiments, the therapy catheter incorporates one or more solid-state thermoelectric cooling devices, such as Peltier devices.

According to various embodiments, a therapy catheter is placed in the renal artery and cools the artery tissue to a temperature at which ice particles form in the artery tissue. The therapy device preferably lowers the temperature to mildly freeze the artery wall, such as to about -10°C. The temperature is controlled so that the region of frozen tissue includes at least the inner portion of the renal artery wall tissue (e.g., the endothelium, media, and inner portion of the adventitia). Preferably, the temperature is controlled so that the region of frozen tissue includes renal artery wall tissue other than that which includes renal sympathetic nerves. The temperature is further controlled so that the outer renal artery wall and perivascular tissue that includes renal sympathetic nerves and ganglia is unfrozen. The lumen-facing section of the cooling arrangement of the therapy catheter is preferably lined with insulation to reduce or prevent freezing of arterial blood, which may be diverted through a diversion arrangement of the catheter.

Microwave energy radiation is transmitted through the frozen artery wall tissue and readily absorbed by the unfrozen innervated tissues of the outer renal artery wall and perivascular space. In some embodiments, ablating unfrozen innervated perivascular renal nerve tissue using microwave energy radiation passing through frozen renal artery wall tissue involves application of approximately 20 Watts of emitted microwave power for approximately 2 minutes to achieve a temperature within the unfrozen innervated perivascular renal nerve tissue of at least 50° C. It is noted that, for the microwave emitter, smaller transmission lines and higher frequencies have higher attenuation. As such, it may be advantageous to emit microwave radiation having a frequency of about 900 MHz rather than about 2.4GHz. In either case, the primary factor impacting the efficacy of the microwave emitter is the difference between ice (frozen tissue) and unfrozen tissue absorption of electromagnetic energy at different frequencies. In some embodiments, the temperature of the cooling apparatus can be lowered during ablation to compensate for the additional heat generated in the tissues subject to microwave energy radiation and/or to cool the transmission line of the microwave emitter.

Various techniques for monitoring the boundary between frozen and unfrozen tissue can be used, including ultrasound scanning in A-mode, B-mode, or M-mode, for example. Other monitoring approaches include use of an opto-acoustic sensor, which incorporates a laser arrangement along with an ultrasound arrangement, in addition to the microwave and cryothermal components of the therapy catheter. Monitoring a change in microwave impedance or a change in a ratio of microwave impedances at different frequencies can be implemented to effect monitoring of the boundary between frozen and unfrozen tissue. Also, determining the amount of heat extracted by the cooling apparatus using well known heat transfer equations can be used to estimate the depth of frozen tissue (e.g., heat transfer coefficient = (Q.coolant / ΔT); ΔT = 37 - T of external cooling apparatus; and Q.coolant = heat extracted by cooling apparatus).

Upon completing the ablation procedure for one of the patient's renal arteries, the contralateral renal artery may be innervated in the same manner. After completing the ablation procedure for both renal arteries, the therapy catheter is removed from the patient's body.

In applications involving relatively thin tissues that are subject to freezing, surrounding tissue and body fluid (e.g., blood) is sufficient to warm the frozen tissue to body temperature. In applications involving relatively thick tissues that are subject to freezing (e.g., organ tissue), it may be desirable to warm such tissue using warmed thermal transfer fluid delivered to the cooling apparatus.

According to some embodiments, the therapy catheter supports both the cooling apparatus and the microwave emitter. For example, a housing for the therapy device is mounted at a distal end of the therapy catheter. The housing encompasses both the cooling arrangement and the microwave emitter, which provides for a relatively low profile configuration. The housing preferably incorporates an expandable structure that can transform between a low-profile introduction configuration and a larger-profile deployed configuration. The low-profile introduction configuration allows the therapy catheter to be readily advanced through the venous or arterial system to a desired body location, for example. The larger-profile deployed configuration allows the therapy catheter to be stabilized at the desired body location, such as within the renal artery. In various embodiments, the expandable structure comprises a balloon, such as a cryoballoon.

In accordance with other embodiments, a therapy catheter arrangement includes a cooling catheter and a separate therapy delivery catheter. The cooling catheter includes a cooling apparatus of a type described herein provided at its distal end. The separate therapy delivery catheter includes a microwave emitter apparatus of a type described herein provided at its distal end. In some applications, the cooling and therapy delivery catheters can be deployed together or separately and delivered to a common location. In other applications, the cooling catheter can be deployed to a location proximate the tissue to be protected and the therapy delivery catheter can be deployed at a separate location near the target tissue to be ablated.

In scenarios where the microwave energy radiation first passes through the frozen non-targeted tissue on route to impinging on the target tissue, freezing of the non-target tissue provides both thermal protection of the non-target tissue and a reduced absorption path through which the microwave energy radiation readily propagates. In scenarios where the microwave energy radiation first passes through the unfrozen target tissue, freezing of the non-target tissue is less critical, since most of the microwave energy radiation is absorbed by the target tissue, and the residual microwave energy radiation passes through the frozen non-targeted tissue. In such scenarios, it may not be necessary to freeze the non-targeted tissue due to the reduced amount of microwave energy radiation impinging on the non-targeted tissue, such that above-freezing cooling may adequately protect the non-targeted tissue. As previously discussed, ultrasound or other imaging or measuring apparatuses can be used to assess the temperature of non-targeted and targeted tissues. For example, an ultrasound or other imaging device can be used to measure the depth of ice formation. Thermocouples or other temperature sensors can be incorporated at the distal tip region of the therapy and/or cooling catheter in accordance with various embodiments.

Various embodiments of the disclosure are directed to apparatuses and methods for renal denervation for treating hypertension. Hypertension is a chronic medical condition in which the blood pressure is elevated. Persistent hypertension is a significant risk factor associated with a variety of adverse medical conditions, including heart attacks, heart failure, arterial aneurysms, and strokes. Persistent hypertension is a leading cause of chronic renal failure. Hyperactivity of the sympathetic nervous system serving the kidneys is associated with hypertension and its progression. Deactivation of nerves in the kidneys via renal denervation can reduce blood pressure, and may be a viable treatment option for many patients with hypertension who do not respond to conventional drugs.

The kidneys are instrumental in a number of body processes, including blood filtration, regulation of fluid balance, blood pressure control, electrolyte balance, and hormone production. One primary function of the kidneys is to remove toxins, mineral salts, and water from the blood to form urine. The kidneys receive about 20-25% of cardiac output through the renal arteries that branch left and right from the abdominal aorta, entering each kidney at the concave surface of the kidneys, the renal hilum.

Blood flows into the kidneys through the renal artery and the afferent arteriole, entering the filtration portion of the kidney, the renal corpuscle. The renal corpuscle is composed of the glomerulus, a thicket of capillaries, surrounded by a fluid-filled, cup-like sac called Bowman's capsule. Solutes in the blood are filtered through the very thin capillary walls of the glomerulus due to the pressure gradient that exists between the blood in the capillaries and the fluid in the Bowman's capsule. The pressure gradient is controlled by the contraction or dilation of the arterioles. After filtration occurs, the filtered blood moves through the efferent arteriole and the peritubular capillaries, converging in the interlobular veins, and finally exiting the kidney through the renal vein.

Particles and fluid filtered from the blood move from the Bowman's capsule through a number of tubules to a collecting duct. Urine is formed in the collecting duct and then exits through the ureter and bladder. The tubules are surrounded by the peritubular capillaries (containing the filtered blood). As the filtrate moves through the tubules and toward the collecting duct, nutrients, water, and electrolytes, such as sodium and chloride, are reabsorbed into the blood.

The kidneys are innervated by the renal plexus which emanates primarily from the aorticorenal ganglion. Renal ganglia are formed by the nerves of the renal plexus as the nerves follow along the course of the renal artery and into the kidney. The renal nerves are part of the autonomic nervous system which includes sympathetic and parasympathetic components. The sympathetic nervous system is known to be the system that provides the bodies "fight or flight" response, whereas the parasympathetic nervous system provides the "rest and digest" response. Stimulation of sympathetic nerve activity triggers the sympathetic response which causes the kidneys to increase production of hormones that increase vasoconstriction and fluid retention. This process is referred to as the renin-angiotensin-aldosterone-system (RAAS) response to increased renal sympathetic nerve activity.

In response to a reduction in blood volume, the kidneys secrete renin, which stimulates the production of angiotensin. Angiotensin causes blood vessels to constrict, resulting in increased blood pressure, and also stimulates the secretion of the hormone aldosterone from the adrenal cortex. Aldosterone causes the tubules of the kidneys to increase the reabsorption of sodium and water, which increases the volume of fluid in the body and blood pressure.

Congestive heart failure (CHF) is a condition that has been linked to kidney function. CHF occurs when the heart is unable to pump blood effectively throughout the body. When blood flow drops, renal function degrades because of insufficient perfusion of the blood within the renal corpuscles. The decreased blood flow to the kidneys triggers an increase in sympathetic nervous system activity (i.e., the RAAS becomes too active) that causes the kidneys to secrete hormones that increase fluid retention and vasorestriction. Fluid retention and vasorestriction in turn increases the peripheral resistance of the circulatory system, placing an even greater load on the heart, which diminishes blood flow further. If the deterioration in cardiac and renal functioning continues, eventually the body becomes overwhelmed, and an episode of heart failure decompensation occurs, often leading to hospitalization of the patient.

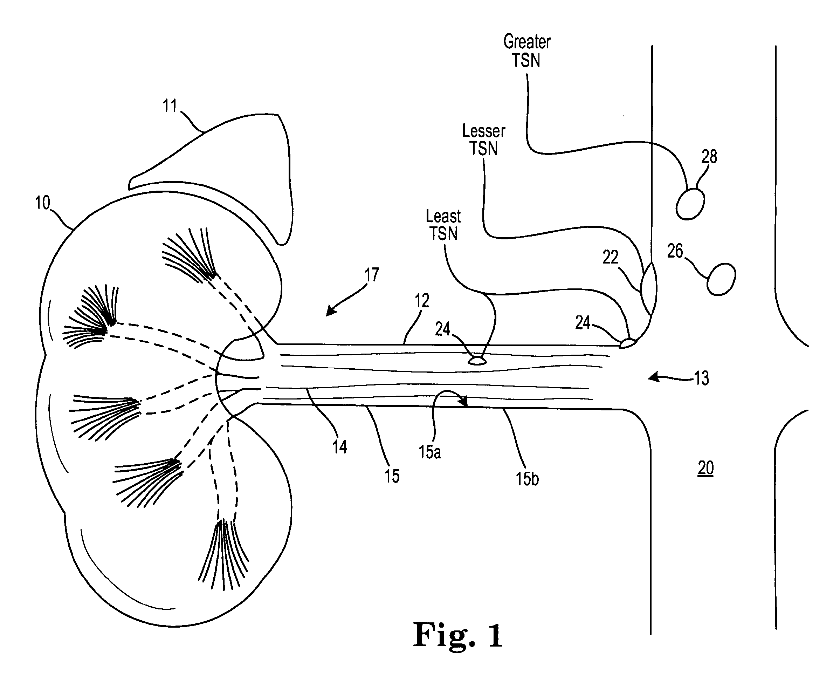

Figure 1 is an illustration of a right kidney 10 and renal vasculature including a renal artery 12 branching laterally from the abdominal aorta 20. In Figure 1, only the right kidney 10 is shown for purposes of simplicity of explanation, but reference will be made herein to both right and left kidneys and associated renal vasculature and nervous system structures, all of which are contemplated within the context of embodiments of the disclosure. The renal artery 12 is purposefully shown to be disproportionately larger than the right kidney 10 and abdominal aorta 20 in order to facilitate discussion of various features and embodiments of the present disclosure.

The right and left kidneys are supplied with blood from the right and left renal arteries that branch from respective right and left lateral surfaces of the abdominal aorta 20. Each of the right and left renal arteries is directed across the crus of the diaphragm, so as to form nearly a right angle with the abdominal aorta 20. The right and left renal arteries extend generally from the abdominal aorta 20 to respective renal sinuses proximate the hilum 17 of the kidneys, and branch into segmental arteries and then interlobular arteries within the kidney 10. The interlobular arteries radiate outward, penetrating the renal capsule and extending through the renal columns between the renal pyramids. Typically, the kidneys receive about 20% of total cardiac output which, for normal persons, represents about 1200 mL of blood flow through the kidneys per minute.

The primary function of the kidneys is to maintain water and electrolyte balance for the body by controlling the production and concentration of urine. In producing urine, the kidneys excrete wastes such as urea and ammonium. The kidneys also control reabsorption of glucose and amino acids, and are important in the production of hormones including vitamin D, renin and erythropoietin.

An important secondary function of the kidneys is to control metabolic homeostasis of the body. Controlling hemostatic functions include regulating electrolytes, acid-base balance, and blood pressure. For example, the kidneys are responsible for regulating blood volume and pressure by adjusting volume of water lost in the urine and releasing erythropoietin and renin, for example. The kidneys also regulate plasma ion concentrations (e.g., sodium, potassium, chloride ions, and calcium ion levels) by controlling the quantities lost in the urine and the synthesis of calcitrol. Other hemostatic functions controlled by the kidneys include stabilizing blood pH by controlling loss of hydrogen and bicarbonate ions in the urine, conserving valuable nutrients by preventing their excretion, and assisting the liver with detoxification.

Also shown in Figure 1 is the right suprarenal gland 11, commonly referred to as the right adrenal gland. The suprarenal gland 11 is a star-shaped endocrine gland that rests on top of the kidney 10. The primary function of the suprarenal glands (left and right) is to regulate the stress response of the body through the synthesis of corticosteroids and catecholamines, including cortisol and adrenaline (epinephrine), respectively. Encompassing the kidneys 10, suprarenal glands 11, renal vessels 12, and adjacent perirenal fat is the renal fascia, e.g., Gerota's fascia, (not shown), which is a fascial pouch derived from extraperitoneal connective tissue.

The autonomic nervous system of the body controls involuntary actions of the smooth muscles in blood vessels, the digestive system, heart, and glands. The autonomic nervous system is divided into the sympathetic nervous system and the parasympathetic nervous system. In general terms, the parasympathetic nervous system prepares the body for rest by lowering heart rate, lowering blood pressure, and stimulating digestion. The sympathetic nervous system effectuates the body's fight-or-flight response by increasing heart rate, increasing blood pressure, and increasing metabolism.

In the autonomic nervous system, fibers originating from the central nervous system and extending to the various ganglia are referred to as preganglionic fibers, while those extending from the ganglia to the effector organ are referred to as postganglionic fibers. Activation of the sympathetic nervous system is effected through the release of adrenaline (epinephrine) and to a lesser extent norepinephrine from the suprarenal glands 11. This release of adrenaline is triggered by the neurotransmitter acetylcholine released from preganglionic sympathetic nerves.

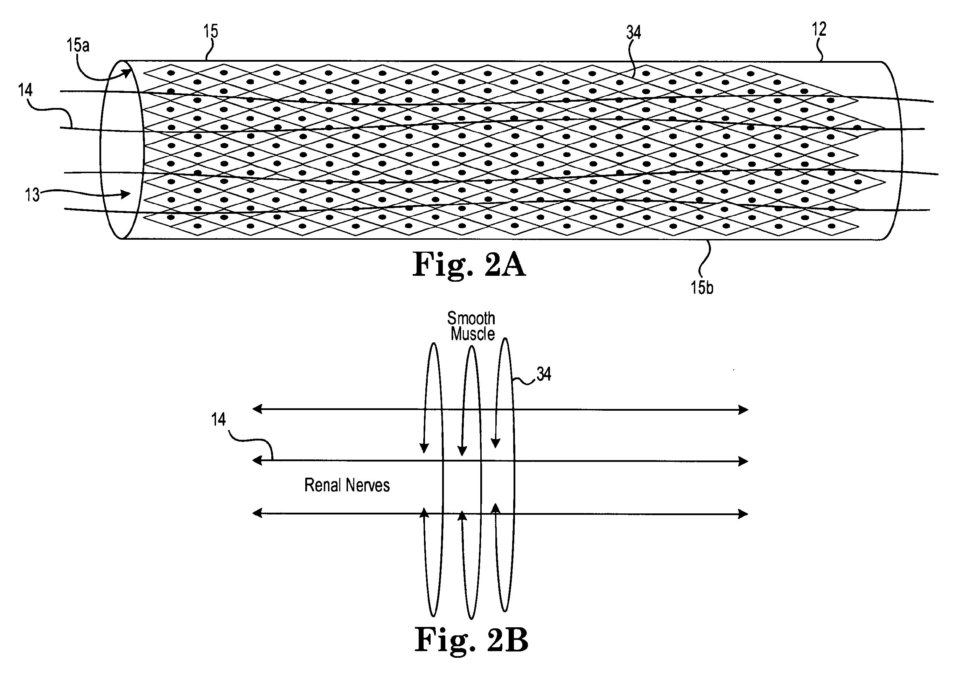

The kidneys and ureters (not shown) are innervated by the renal nerves 14. Figures 1 and 2A-2B illustrate sympathetic innervation of the renal vasculature, primarily innervation of the renal artery 12. The primary functions of sympathetic innervation of the renal vasculature include regulation of renal blood flow and pressure, stimulation of renin release, and direct stimulation of water and sodium ion reabsorption.

Most of the nerves innervating the renal vasculature are sympathetic postganglionic fibers arising from the superior mesenteric ganglion 26. The renal nerves 14 extend generally axially along the renal arteries 12, enter the kidneys 10 at the hilum 17, follow the branches of the renal arteries 12 within the kidney 10, and extend to individual nephrons. Other renal ganglia, such as the renal ganglia 24, superior mesenteric ganglion 26, the left and right aorticorenal ganglia 22, and celiac ganglia 28 also innervate the renal vasculature. The celiac ganglion 28 is joined by the greater thoracic splanchnic nerve (greater TSN). The aorticorenal ganglia 26 is joined by the lesser thoracic splanchnic nerve (lesser TSN) and innervates the greater part of the renal plexus.

Sympathetic signals to the kidney 10 are communicated via innervated renal vasculature that originates primarily at spinal segments T10-T12 and L1. Parasympathetic signals originate primarily at spinal segments S2-S4 and from the medulla oblongata of the lower brain. Sympathetic nerve traffic travels through the sympathetic trunk ganglia, where some may synapse, while others synapse at the aorticorenal ganglion 22 (via the lesser thoracic splanchnic nerve, i.e., lesser TSN) and the renal ganglion 24 (via the least thoracic splanchnic nerve, i.e., least TSN). The postsynaptic sympathetic signals then travel along nerves 14 of the renal artery 12 to the kidney 10. Presynaptic parasympathetic signals travel to sites near the kidney 10 before they synapse on or near the kidney 10.

With particular reference to Figure 2A, the renal artery 12, as with most arteries and arterioles, is lined with smooth muscle 34 that controls the diameter of the renal artery lumen 13. Smooth muscle, in general, is an involuntary non-striated muscle found within the media layer of large and small arteries and veins, as well as various organs. The glomeruli of the kidneys, for example, contain a smooth muscle-like cell called the mesangial cell. Smooth muscle is fundamentally different from skeletal muscle and cardiac muscle in terms of structure, function, excitation-contraction coupling, and mechanism of contraction.

Smooth muscle cells can be stimulated to contract or relax by the autonomic nervous system, but can also react on stimuli from neighboring cells and in response to hormones and blood borne electrolytes and agents (e.g., vasodilators or vasoconstrictors). Specialized smooth muscle cells within the afferent arteriole of the juxtaglomerular apparatus of kidney 10, for example, produces renin which activates the angiotension II system.

The renal nerves 14 innervate the smooth muscle 34 of the renal artery wall 15 and extend lengthwise in a generally axial or longitudinal manner along the renal artery wall 15. The smooth muscle 34 surrounds the renal artery circumferentially, and extends lengthwise in a direction generally transverse to the longitudinal orientation of the renal nerves 14, as is depicted in Figure 2B.

The smooth muscle 34 of the renal artery 12 is under involuntary control of the autonomic nervous system. An increase in sympathetic activity, for example, tends to contract the smooth muscle 34, which reduces the diameter of the renal artery lumen 13 and decreases blood perfusion. A decrease in sympathetic activity tends to cause the smooth muscle 34 to relax, resulting in vessel dilation and an increase in the renal artery lumen diameter and blood perfusion. Conversely, increased parasympathetic activity tends to relax the smooth muscle 34, while decreased parasympathetic activity tends to cause smooth muscle contraction.

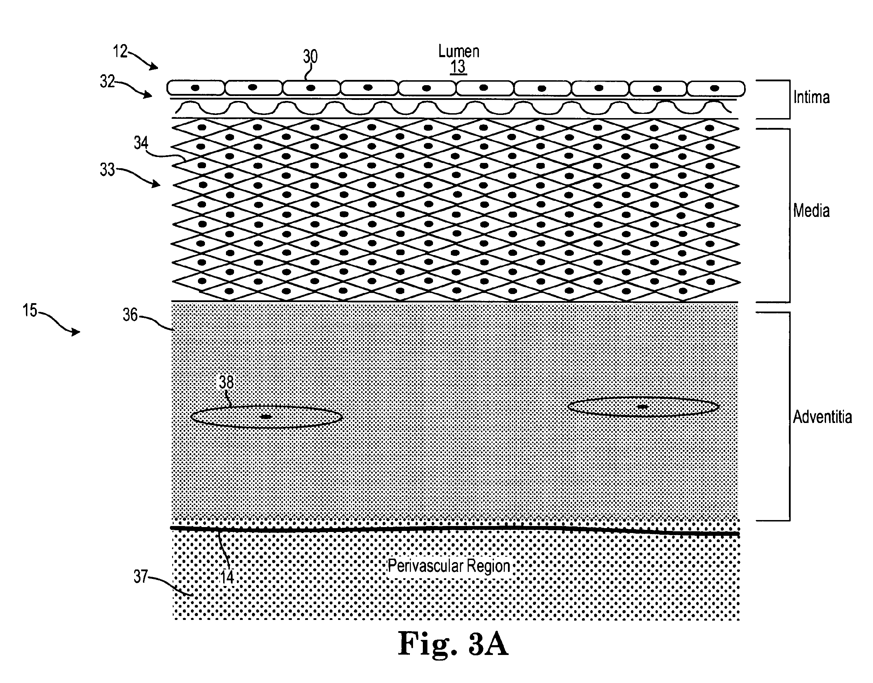

Figure 3A shows a segment of a longitudinal cross-section through a renal artery, and illustrates various tissue layers of the wall 15 of the renal artery 12. The innermost layer of the renal artery 12 is the endothelium 30, which is the innermost layer of the intima 32 and is supported by an internal elastic membrane. The endothelium 30 is a single layer of cells that contacts the blood flowing though the vessel lumen 13. Endothelium cells are typically polygonal, oval, or fusiform, and have very distinct round or oval nuclei. Cells of the endothelium 30 are involved in several vascular functions, including control of blood pressure by way of vasoconstriction and vasodilation, blood clotting, and acting as a barrier layer between contents within the lumen 13 and surrounding tissue, such as the membrane of the intima 32 separating the intima 32 from the media 34, and the adventitia 36. The membrane or maceration of the intima 32 is a fine, transparent, colorless structure which is highly elastic, and commonly has a longitudinal corrugated pattern.

Adjacent the intima 32 is the media 33, which is the middle layer of the renal artery 12. The media is made up of smooth muscle 34 and elastic tissue. The media 33 can be readily identified by its color and by the transverse arrangement of its fibers. More particularly, the media 33 consists principally of bundles of smooth muscle fibers 34 arranged in a thin plate-like manner or lamellae and disposed circularly around the arterial wall 15. The outermost layer of the renal artery wall 15 is the adventitia 36, which is made up of connective tissue. The adventitia 36 includes fibroblast cells 38 that play an important role in wound healing.

A perivascular region 37 is shown adjacent and peripheral to the adventitia 36 of the renal artery wall 15. A renal nerve 14 is shown proximate the adventitia 36 and passing through a portion of the perivascular region 37. The renal nerve 14 is shown extending substantially longitudinally along the outer wall 15 of the renal artery 12. The main trunk of the renal nerves 14 generally lies in or on the adventitia 36 of the renal artery 12, often passing through the perivascular region 37, with certain branches coursing into the media 33 to enervate the renal artery smooth muscle 34.

Embodiments of the disclosure may be implemented to provide varying degrees of denervation therapy to innervated renal vasculature. For example, embodiments of the disclosure may provide for control of the extent and relative permanency of renal nerve impulse transmission interruption achieved by denervation therapy delivered using a treatment apparatus of the disclosure. The extent and relative permanency of renal nerve injury may be tailored to achieve a desired reduction in sympathetic nerve activity (including a partial or complete block) and to achieve a desired degree of permanency (including temporary or irreversible injury).

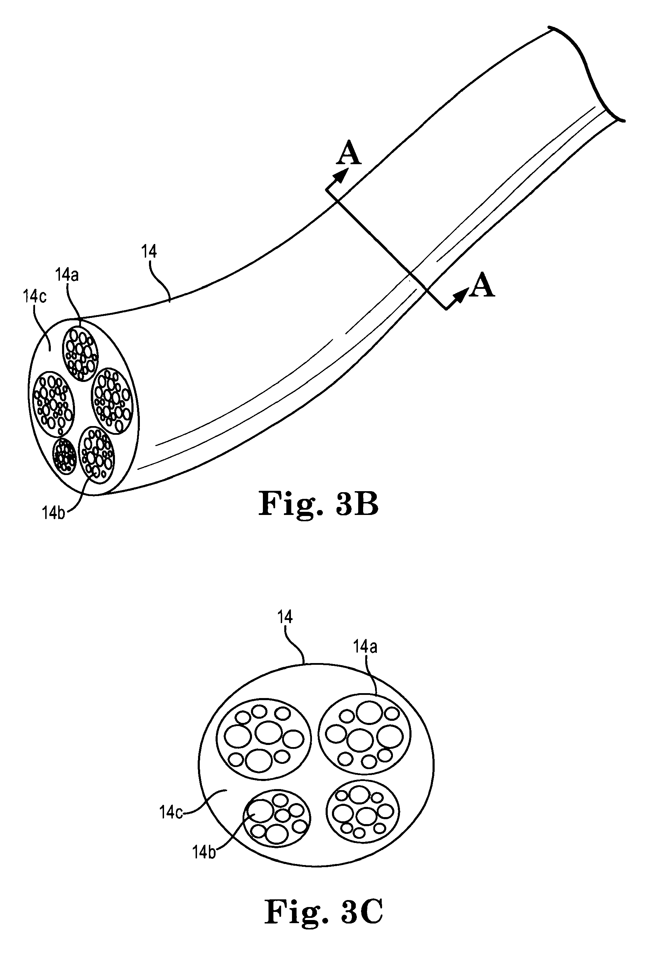

Returning to Figures 3B and 3C, the portion of the renal nerve 14 shown in Figures 3B and 3C includes bundles 14a of nerve fibers 14b each comprising axons or dendrites that originate or terminate on cell bodies or neurons located in ganglia or on the spinal cord, or in the brain. Supporting tissue structures 14c of the nerve 14 include the endoneurium (surrounding nerve axon fibers), perineurium (surrounds fiber groups to form a fascicle), and epineurium (binds fascicles into nerves), which serve to separate and support nerve fibers 14b and bundles 14a. In particular, the endoneurium, also referred to as the endoneurium tube or tubule, is a layer of delicate connective tissue that encloses the myelin sheath of a nerve fiber 14b within a fasciculus.

Major components of a neuron include the soma, which is the central part of the neuron that includes the nucleus, cellular extensions called dendrites, and axons, which are cable-like projections that carry nerve signals. The axon terminal contains synapses, which are specialized structures where neurotransmitter chemicals are released in order to communicate with target tissues. The axons of many neurons of the peripheral nervous system are sheathed in myelin, which is formed by a type of glial cell known as Schwann cells. The myelinating Schwann cells are wrapped around the axon, leaving the axolemma relatively uncovered at regularly spaced nodes, called nodes of Ranvier. Myelination of axons enables an especially rapid mode of electrical impulse propagation called saltation.

In some embodiments, a treatment apparatus of the disclosure may be implemented to deliver denervation therapy that causes transient and reversible injury to renal nerve fibers 14b. In other embodiments, a treatment apparatus of the disclosure may be implemented to deliver denervation therapy that causes more severe injury to renal nerve fibers 14b, which may be reversible if the therapy is terminated in a timely manner. In preferred embodiments, a treatment apparatus of the disclosure may be implemented to deliver denervation therapy that causes severe and irreversible injury to renal nerve fibers 14b, resulting in permanent cessation of renal sympathetic nerve activity. For example, a treatment apparatus may be implemented to deliver a denervation therapy that disrupts nerve fiber morphology to a degree sufficient to physically separate the endoneurium tube of the nerve fiber 14b, which can prevent regeneration and re-innervation processes.

By way of example, and in accordance with Seddon's classification as is known in the art, a treatment apparatus of the disclosure may be implemented to deliver a denervation therapy that interrupts conduction of nerve impulses along the renal nerve fibers 14b by imparting damage to the renal nerve fibers 14b consistent with neruapraxia. Neurapraxia describes nerve damage in which there is no disruption of the nerve fiber 14b or its sheath. In this case, there is an interruption in conduction of the nerve impulse down the nerve fiber, with recovery taking place within hours to months without true regeneration, as Wallerian degeneration does not occur. Wallerian degeneration refers to a process in which the part of the axon separated from the neuron's cell nucleus degenerates. This process is also known as anterograde degeneration. Neurapraxia is the mildest form of nerve injury that may be imparted to renal nerve fibers 14b by use of a treatment apparatus according to embodiments of the disclosure.

A treatment apparatus may be implemented to interrupt conduction of nerve impulses along the renal nerve fibers 14b by imparting damage to the renal nerve fibers consistent with axonotmesis. Axonotmesis involves loss of the relative continuity of the axon of a nerve fiber and its covering of myelin, but preservation of the connective tissue framework of the nerve fiber. In this case, the encapsulating support tissue 14c of the nerve fiber 14b are preserved. Because axonal continuity is lost, Wallerian degeneration occurs. Recovery from axonotmesis occurs only through regeneration of the axons, a process requiring time on the order of several weeks or months. Electrically, the nerve fiber 14b shows rapid and complete degeneration. Regeneration and re-innervation may occur as long as the endoneural tubes are intact.

A treatment apparatus may be implemented to interrupt conduction of nerve impulses along the renal nerve fibers 14b by imparting damage to the renal nerve fibers 14b consistent with neurotmesis. Neurotmesis, according to Seddon's classification, is the most serious nerve injury in the scheme. In this type of injury, both the nerve fiber 14b and the nerve sheath are disrupted. While partial recovery may occur, complete recovery is not possible. Neurotmesis involves loss of continuity of the axon and the encapsulating connective tissue 14c, resulting in a complete loss of autonomic function, in the case of renal nerve fibers 14b. If the nerve fiber 14b has been completely divided, axonal regeneration causes a neuroma to form in the proximal stump.

A more stratified classification of neurotmesis nerve damage may be found by reference to the Sunderland System as is known in the art. The Sunderland System defines five degrees of nerve damage, the first two of which correspond closely with neurapraxia and axonotmesis of Seddon's classification. The latter three Sunderland System classifications describe different levels of neurotmesis nerve damage.

The first and second degrees of nerve injury in the Sunderland system are analogous to Seddon's neurapraxia and axonotmesis, respectively. Third degree nerve injury, according to the Sunderland System, involves disruption of the endoneurium, with the epineurium and perineurium remaining intact. Recovery may range from poor to complete depending on the degree of intrafascicular fibrosis. A fourth degree nerve injury involves interruption of all neural and supporting elements, with the epineurium remaining intact. The nerve is usually enlarged. Fifth degree nerve injury involves complete transection of the nerve fiber 14b with loss of continuity.

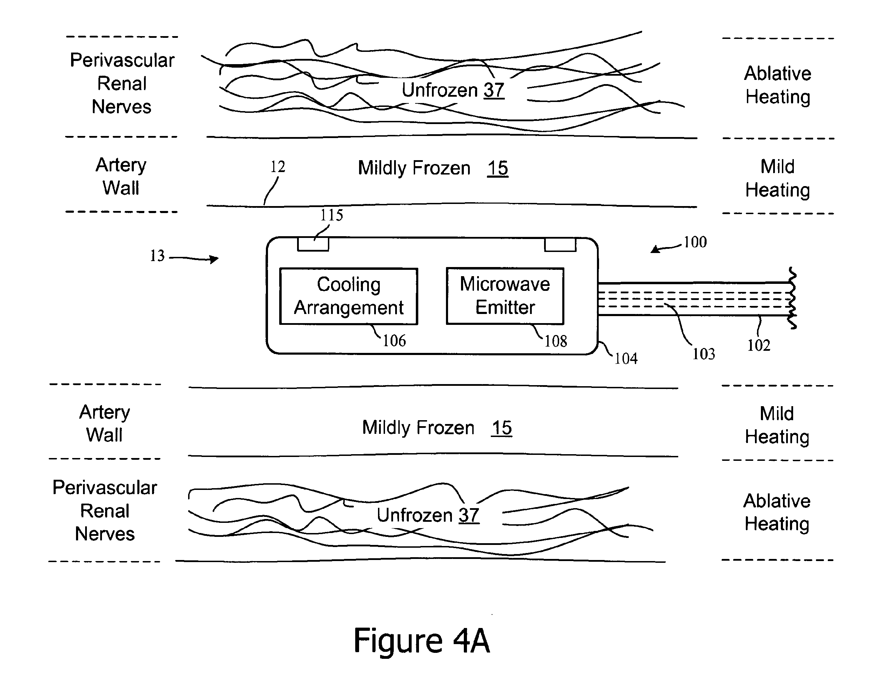

Figure 4A shows an embodiment of the disclosure which includes a therapy catheter 100 configured for placement within the lumen 13 of a patient's renal artery 12. The therapy catheter 100 shown in Figure 4A includes a therapy device 104 provided at a distal end of a shaft 102 of the therapy catheter 100. The therapy device 104 is configured to deliver renal nerve ablation using microwave energy radiating from a microwave emitter 108, with cryothermal cooling provided by a thermal unit 106 of the therapy catheter 100. The thermal unit 106 can include a phase-change cryothermal mechanism, a simpler heat exchanger system with liquid coolant, or a solid-state thermoelectric cooling device, for example. Various cooling elements and support, connection, and control arrangements and methodologies that can be adapted for use in embodiments of the present disclosure are disclosed in commonly owned U.S. Patent No. 7,238,184 and U.S. Patent Application No. 13/157,844 filed June 10, 2011. The microwave emitter 108 and the thermal unit 106 are respectively electrically and fluidly coupled to an external control system via a lumen arrangement 103.

As is illustrates in Figure 4A, the thermal unit 106 is controlled to cause mild freezing of the renal artery wall 15. It is understood that the terms "freezing" and "frozen" within the context of various embodiments refers to the presence of ice particles within the tissue of interest, such as the renal artery wall 15. With the renal artery wall 15 in a mildly frozen state, the microwave emitter 108 is controlled to direct microwave energy into the renal artery wall 15 and adjacent perivascular space 37 which includes sympathetic renal nerves and ganglia. The temperature of the target sympathetic renal nerve tissue, which is in an unfrozen state, is preferably raised to about 55°C or higher in order to ablate the target tissue. During ablation of the target tissue, the non-targeted artery wall tissue 15 remains at or below freezing, which may require additional removal of heat due to microwave heating of the neighboring target tissue.

In some embodiments, a complete circumferential region (a full 360° region) of the target sympathetic renal nerve tissue can be ablated concurrently. In other embodiments, less than a complete circumferential region of the target sympathetic renal nerve tissue can be ablated. As will be discussed hereinbelow, the microwave emitter 108 can be configured to emit an omni-directional, unidirectional, or a focused radiation pattern.

One or more temperature sensors 115 can be situated on the therapy device 104 to provide for temperature sensing at the renal artery wall 15. In some embodiments, a temperature sensor 115 can be implemented as a temperature probe that is advanced against or into the renal artery wall tissue. For example, a temperatures sensor 115 can be situated on a tissue piercing member that is extendable from the therapy device 104 (or other location at the distal end of the shaft 102). During an ablation procedure, the tissue piercing member can be advanced from a location within or on the therapy device 104 and into the renal artery wall 15. The temperature sensor 115 can be used to sense the local temperature within the renal artery wall 15 to verify that the artery wall tissue is mildly frozen. The temperature sensor 115 can also be advanced into the adjacent perivascular space 37 to verify that perivascular renal artery tissue included within the perivascular tissue is unfrozen. The temperature sensor 115 can be moved between artery wall tissue and perivascular tissue to provide local temperature and changes thereto during ablation of the perivascular tissue (and outer renal artery wall tissue if appropriate).

In other configurations, an extendable non-tissue penetrating (e.g., blunt or bulbous ended) member may be used to move a temperature sensor 115 into contact with the renal artery wall 15. Forcing the temperature sensor 115 against the renal artery wall 15 compresses the artery wall which can provide a good estimate of actual renal artery wall temperature. Based on this temperature and clinical data on thermal transfer properties of artery wall tissue and perivascular nerve tissue, the temperature of the adjacent perivascular tissue can be estimated.

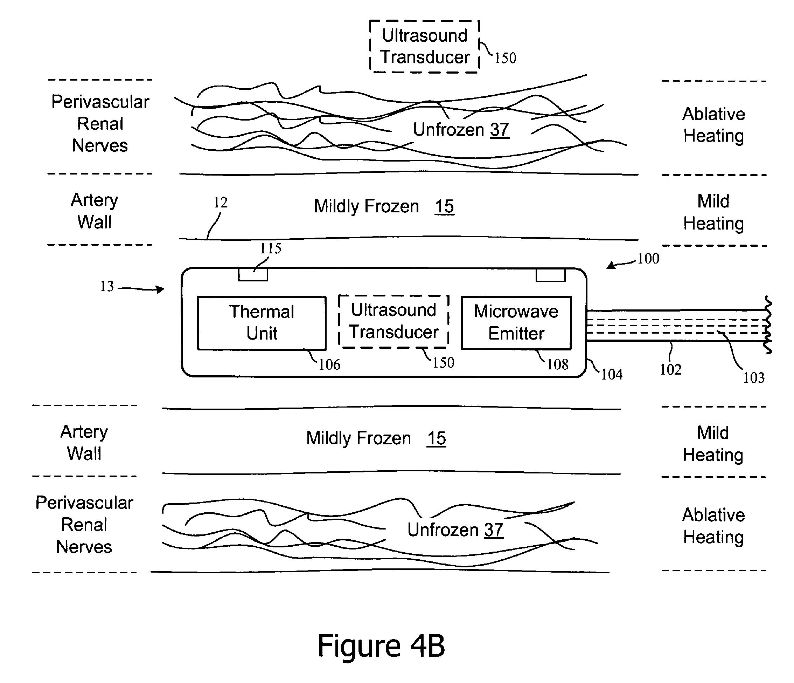

Figure 4B illustrates another embodiment of a therapy catheter 100 that incorporates an ultrasound transducer 150. The ultrasound transducer 150 is preferably configured to operate in a mode (A-, B-, or M-ultrasound mode) that facilitates detection of ice within tissue as well as determining the depth of such ice. The ultrasound transducer 150 is preferably operable to distinguish between frozen and unfrozen tissue. In the embodiment shown in Figure 4B, the ultrasound transducer 150 is integrated as a component of the therapy device 104. It is understood that the ultrasound transducer 150 may be provided on a separate catheter that can be positioned within the renal artery 12 or at other body locations, such as a renal vein, ureter, or other nearby vessel. The ultrasound transducer 150 may also be configured as a patient's-external device.

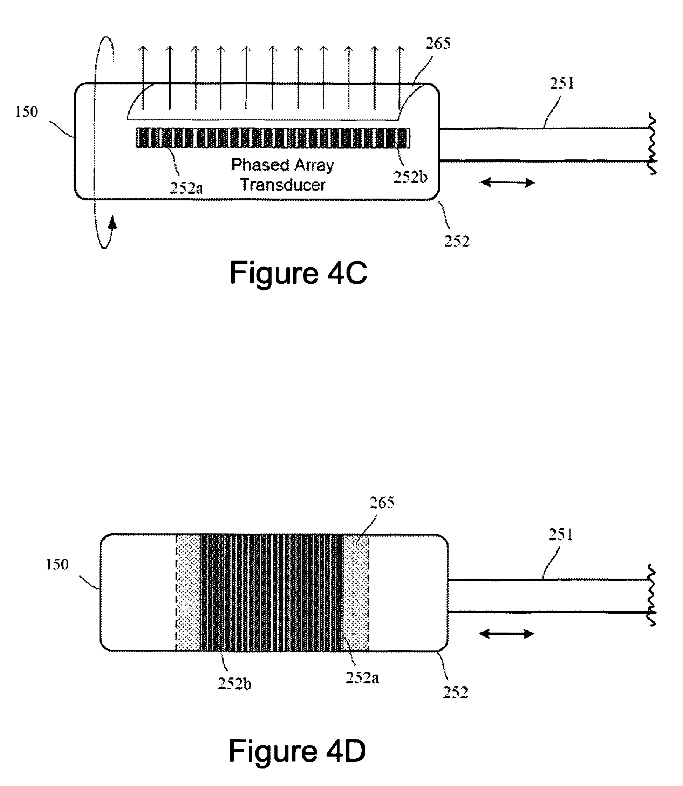

In the embodiment shown in Figures 4C and 4D, the ultrasound transducers 150 are shown as provided at a distal end of a catheter 251, rather than integrated in the therapy device 104 as shown in Figure 4B. It is understood that the ultrasound transducers 150 shown in Figures 4C and 4D can also be integrated into the therapy device 104. Referring to Figure 4C, an emitter 252 of the ultrasound unit 150 includes an acoustic phased array transducer 252a which comprises a multiplicity of acoustic elements 252b. The phased array transducer 252a shown in Figure 4C extends over a radial segment of the ultrasound unit's circumference, allowing an acoustic energy beam 262 to pass through an aperture 265 (e.g., focusing lens arrangement) and impinge on target tissue. The emitter 252 of the ultrasound unit 250 may be aimed at target tissue by rotating and translating the catheter 251 (i.e., the body of the medical device) or by moving the ultrasound unit 250 relative to the catheter 251, either manually or robotically.

In the embodiment shown in Figure 4D, a phased array transducer 252a extends over all or nearly all of the ultrasound unit's circumference, allowing an acoustic energy beam 262 to pass through an annular aperture 265 (e.g., focusing lens arrangement) and impinge on a circular or cylindrical target tissue region. This embodiment of ultrasound unit 150 is particularly useful when imaging an entire circumferential region of the renal artery and perivascular tissue without having to translate or rotate the catheter 251 or ultrasound unit 150.

The ultrasound unit 150 preferably has a capability that allows for focusing of acoustic energy at desired distances so that all or most of the perivascular space adjacent the outer wall of the renal artery can be subject to imaging. Details of these and other ultrasound apparatuses and methods are described in U.S. Patent Application Serial No. 13/086,116 on April 13, 2011, which claims the benefit of U.S. Provisional Patent Application Serial No. 61/324,164 filed April 14, 2010.

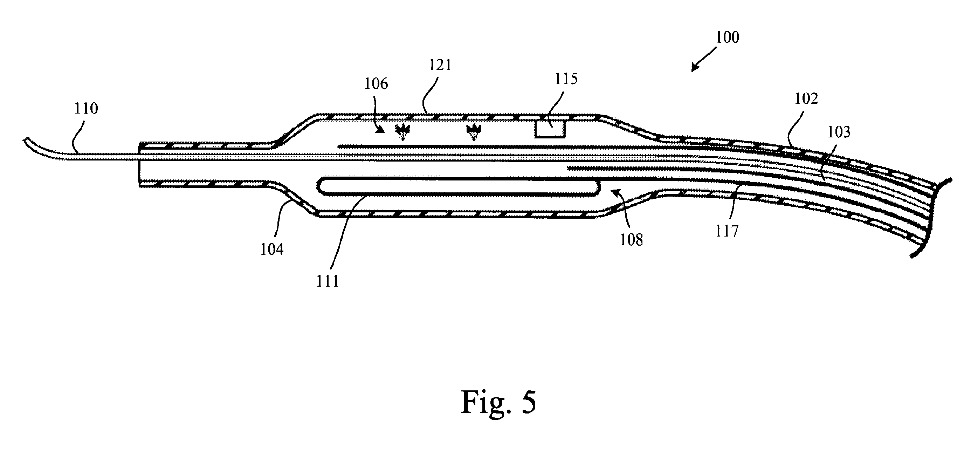

Figure 5 shows a therapy catheter 100 configured for deployment within a patient's renal artery in accordance with various embodiments. The therapy catheter 100 includes a therapy device 104 provided at a distal tip end of a catheter shaft 102. The therapy device 104 is electrically and fluidly coupled to patient-external system via a lumen arrangement 103 which runs along the length of the shaft 102. The therapy device 104 and the lumen arrangement 103 of the shaft 102 may respectively include a lumen dimensioned to receive a guidewire 110. The guidewire 110 can be used by the clinician to access a patient's arterial system, locate the patient's renal artery 12, and advanced the therapy catheter 100 into the lumen 13 of the renal artery 12. In some embodiments, a guide catheter may be used alone or in combination with a guidewire 110 to access the lumen of a patient's renal artery 12. Various known over-the-wire steering techniques may be used for intravascular navigation and deployment of the therapy device 104 within the lumen 13 of the renal artery 12.

The therapy device 104 is shown to include a microwave emitter 108 which comprises an antenna 111. The microwave emitter 108 is electrically coupled to an external microwave energy source via a conductor 117 which runs along the length of the shaft 102. In general, the microwave emitter 108 propagates an electromagnetic field that radiates in a plane perpendicular to the major axis of the antenna 111. In some embodiments, the microwave emitter 108 produces and omni-directional electromagnetic field (e.g., circumferential field pattern) that propagates through a circumferential portion of the renal artery wall 15. The microwave emitter 108 can be implemented so that very little of the electromagnetic field propagates forward of the distal tip of the antenna 111. In other embodiments, the microwave emitter 108 produces a unidirectional or focused electromagnetic field that propagates through a predefined arc portion of the renal artery wall 15. Accordingly, the electromagnetic field produced by the microwave emitter 108 can be shaped and directed in a manner appropriate for a given application.

The therapy unit 104 includes a housing 121 which is typically constructed from polymeric material. The housing 121 preferably has a diameter dimensioned to fit within a renal artery 12 of an average patient. It is understood that different models of ablation catheters 100 can be constructed each having specific housing configurations and dimensions appropriate for a given population of patients. In some embodiments, the housing 121 may comprise an expandable element, such as a pressurizable balloon or a mechanically expandable arrangement. Use of such an expandable element in the construction of the housing 121 allows for use of a common housing design for a population of patients having varying anatomy. In accordance with various embodiments in which a pressurizable balloon is used in the construction of the housing 121, a thermal transfer fluid may be used for pressurizing the balloon and cooling renal artery tissue. A separate pressurizable lumen arrangement can be provided within the catheter shaft 102 for controlling the pressure within the balloon.

It may be desirable to limit the amount of arterial blood that passes between the microwave emitter 108 and renal artery wall 15 in order to limit undesirable effects resulting from blood heating. The housing 121 can be configured to include one or more blood diverging features, such as inlet and outlet ports provided at the proximal and distal ends of the housing 121. Arterial blood can be diverted away from the renal artery wall 15 by channeling arterial blood through the inlet and outlet ports of the housing 121. In configurations that employee a directional microwave emitter 108, the housing 121 may include one or more longitudinal flutes arranged along the portion of housing 121 through which no or negligible electromagnetic field emanates.

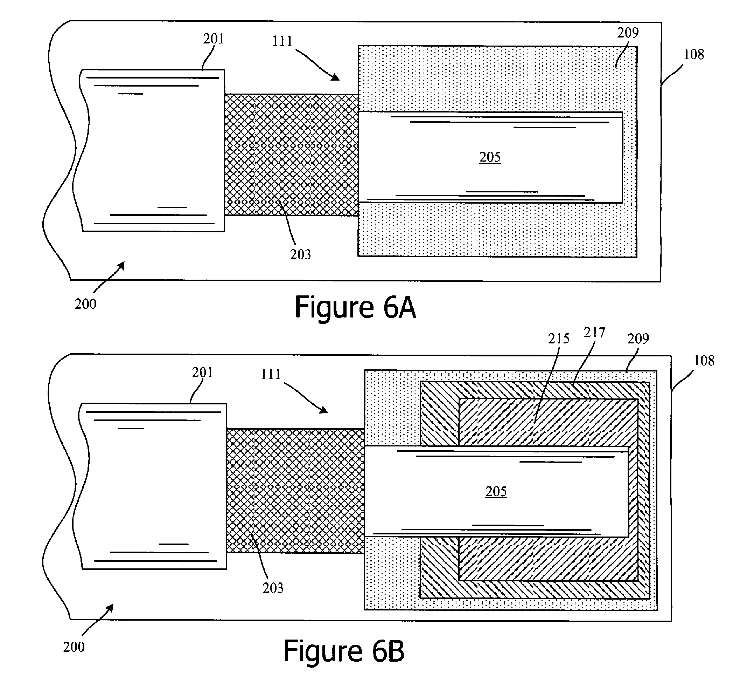

Turning now to Figures 6A and 6B, these figures illustrate different configurations of a microwave emitter 108 that can be incorporated into an ablation catheter in accordance with embodiments of the disclosure. The microwave emitter 108 shown in Figures 6A and 6B represent two of many other possible microwave emitter configurations that can be implemented in an ablation catheter that combines microwave ablation with protective freezing of non-targeted tissue. As such, the particular configuration of the microwave emitter 108 can vary from those shown herein for illustrative purposes. For example, selected features of the microwave catheters disclosed in the following U.S. issue patents can be implemented in various embodiments of a microwave ablation catheter of the present disclosure: U.S. Patent Nos. 6471696, 6699241, 6447505, 5957969, 5904709 and 5364392.

Figure 6A shows an embodiment of a microwave emitter 108 in accordance with various embodiments. The microwave emitter 108 shown in Figure 6A includes a coaxial cable 200 that runs along the length of the catheter shaft 102. Various components at the distal end of the coaxial cable 200 are illustrated, including a sheath 201 which encompasses a metallic shield 203. Disposed within the magnetic shield 203 is a portion of a conductor 205 which defines the microwave energy radiating portion of the antenna 111. An exposed length of the conductor 205 can be embedded within a dielectric compound 209 having low microwave energy loss. The compound 209 preferably provides a high dielectric constant for the antenna 205, and can be fashioned to shape and direct the microwave energy radiation emitted by the antenna 205.

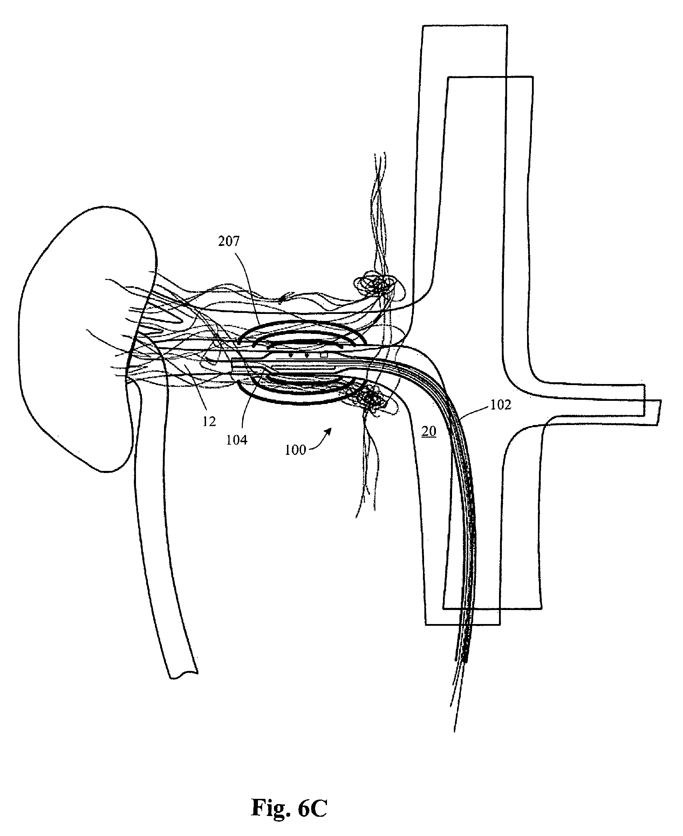

The antenna configuration of the microwave emitter 108 shown in Figure 6A generates a generally circumferential electromagnetic field (e.g., omni-directional about the central axis of the major axis of the conductor 205) that can be propagated from the antenna 205. Figure 6C shows an embodiment of the therapy unit 104 containing an omni-directional microwave emitter 108, such as that shown in Figure 6A, deployed in a patient's renal artery 12 via an access route that includes the inferior aorta 20. Figure 6C shows a generally cylindrically shaped electromagnetic field emanating from the microwave emitter 108 during ablation. It is noted that the intensity pattern of some microwave emitters 108 is more like a toroid than a cylinder in shape, with a very small minor diameter rather than a plane. Alternatively, the intensity pattern may be raindrop shaped, or a raindrop rotated about the axis. In most cases, the intensity pattern is axisymmetric, but slot antennas typically have an off-center, biased intensity pattern.

Figure 6B shows an embodiment of a microwave emitter 108 in accordance with other embodiments. The microwave emitter 108 shown in Figure 6B has a structure similar to that shown in Figure 6A, but is constructed to propagate a unidirectional or directional (e.g., partially circumferential) electromagnetic field. In the embodiment shown in Figure 6B, the conductor portion 205 of the antenna 111 is in contact with a dielectric material which defines a dielectric material plane 215. An energy conducting ground plane 217 is shown in contact with the dielectric material plane 215. The conductor portion 205, dielectric material plane 215, and ground plane 217 are preferably embedded in the dielectric compound 209. The antenna 111 of Figure 6B propagates an electromagnetic field that radiates in a preferred (e.g., single) direction relative to the major axis of the conductor portion 205. Little or only negligible electromagnetic field is emitted radially from the ground plane 217 and the distal tip of the conductor portion 205.

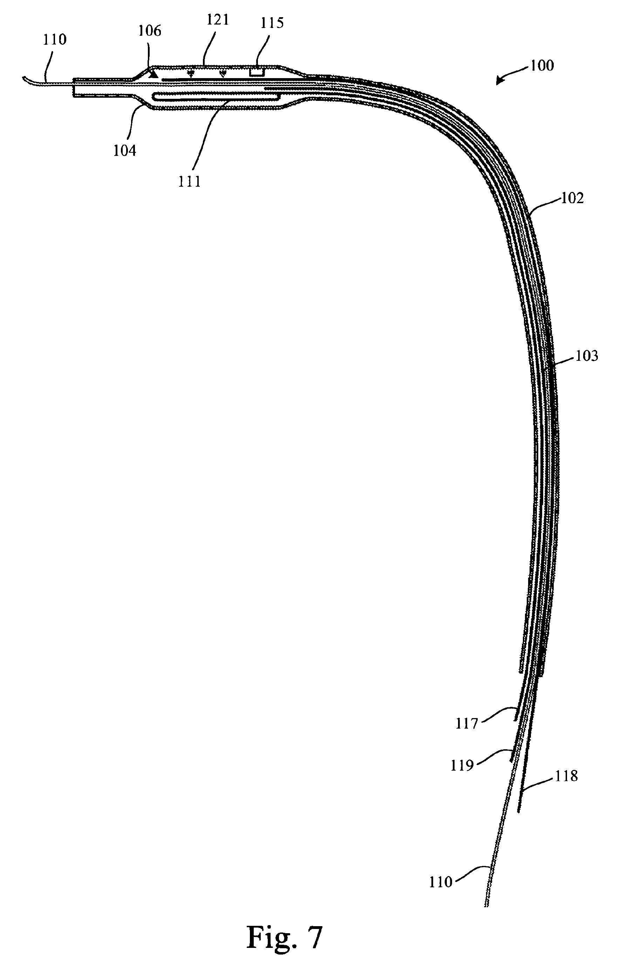

Figure 7 shows details of a lumen arrangement 103 of a therapy catheter 100 in accordance with various embodiments. In the embodiment shown in Figure 7, the lumen arrangement 103 includes a fluid delivery arrangement configured to transport a thermal transfer fluid to and from the distal end the thermal unit 106. The fluid delivery arrangement is fluidly coupled to a fluid source which may be configured to supply a pressurized thermal transfer fluid to the thermal unit 106.

The fluid delivery arrangement shown in Figure 7 includes at least two lumens 118 and 119 configured as supply and return lumens for supplying a cryogen to the thermal unit 106 and returning spent cryogen or gas to the proximal end of the catheter 100, respectively. The cryogen may be circulated through the thermal unit 106 via a hydraulic circuit that includes a cryogen source, supply and return lumens 118, 119, and the thermal unit 106 of the therapy device 104 disposed at the distal end of the catheter 100. The shaft 102 of the catheter 100 is preferably lined with or otherwise incorporates insulation material(s) having appropriate thermal and mechanical characteristics suitable for a selected cryogen.

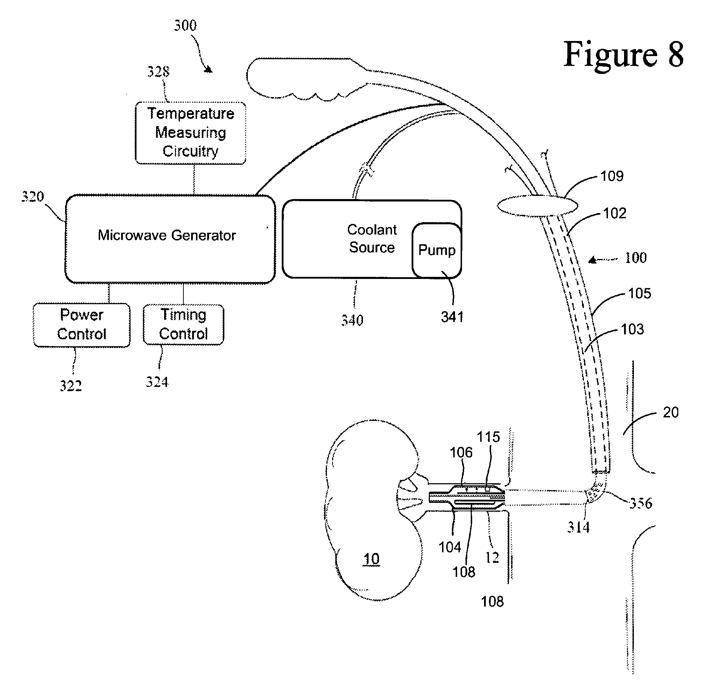

The fluid delivery arrangement is preferably fluidly coupled to a cryogen source which includes a reservoir arrangement fluidly coupled to a pump system, described hereinbelow with reference to Figure 8. A cryogen is contained within the reservoir arrangement. A variety of cryogens may be employed, including cold saline or cold saline and ethanol mixture, Freon or other fluorocarbon refrigerants, nitrous oxide, liquid nitrogen, and liquid carbon dioxide, for example. The fluid delivery arrangement may also be configured to supply a warm thermal transfer fluid to the thermal unit 106, for warming frozen non-targeted tissue. Preferably, warm thermal transfer fluid is communicated to and from the thermal unit 106 using the same fluid delivery arrangement that transports the cryogen to and from the thermal unit 106. Alternatively, the lumen arrangement 103 may include a separate supply and return lumen arrangement for transporting warm thermal transfer fluid to the thermal unit 106 and spent thermal fluid from the thermal unit 106.

In some embodiments, the thermal unit 106 includes a phase-change cooling arrangement. According to various embodiments, a liquid cryogen is supplied to the thermal unit 106 via a supply lumen 118. When released inside the thermal unit 106, the liquid cryogen undergoes a phase change that cools the thermal unit 106 by absorbing the latent heat of vaporization from the tissue surrounding the therapy unit 106, and by cooling of the vaporized gas as it enters a region of lower pressure inside the thermal unit 106 (via the Joule-Thomson effect).

As a result of the phase change and the Joule-Thompson effect, heat is extracted from the surroundings of the therapy unit 106, thereby cooling the renal artery wall to a temperature below freezing. The gas released inside the thermal unit 106 is exhausted through a return lumen 119 provided in the lumen arrangement 103 of shaft 102. The pressure inside the thermal unit 106 may be controlled by regulating one or both of a rate at which cryogen is delivered and a rate at which the exhaust gas is extracted. One or more temperature sensors 115 are preferably provided at the thermal unit 106 (e.g., on the housing 121) for measuring arterial tissue temperature. The lumen arrangement 103 of the catheter shaft 102 includes one or more conductors for coupling each of the temperature sensors 115 to an external temperature unit. The lumen arrangement 103 also includes a microwave conductor 117 coupled to the microwave emitter 108 and an external microwave energy source. As such, the intensity of the electromagnetic field propagating from the microwave emitter 108 can be regulated by the external microwave energy source during the ablation.

Referring now to Figure 8, there is shown a system 300 for ablating tissue that influences sympathetic renal nerve activity in accordance with various embodiments. The system 300 shown in Figure 7 includes a therapy device 104 provided at the distal end of a therapy catheter 100 deployed within a patient's renal artery 12. The therapy catheter 100 includes a flexible shaft 102 within which a lumen arrangement 103 is provided. The shaft 102 is preferably sufficient in length to reach a patient's renal artery 12 from a percutaneous access location 109. It may be desirable to use an external sheath 105 to facilitate delivery of the therapy device 104 into the renal artery 12. The catheter shaft 102 may include a distal hinge 356 that facilitates navigation of a near 90° turn into the renal artery 12 from the aorta 20.

The therapy device 104 includes a thermal unit 106 and a microwave emitter 108 of a type previously described. The microwave emitter 108 of the therapy catheter 100 is electrically coupled to an external microwave generator 320. A power control 322 and timing control 324 provide for automatic or semi-automatic control of microwave energy emission from the therapy unit 104. The thermal unit 106 is shown fluidly coupled to a coolant source 340. A temperature control 324 is preferably coupled to one or more temperature sensors 115 provided at the therapy device 104. The temperature control 324 generates temperature signals which are used by the microwave generator 320 and coolant source 340 to adjust (automatically via a processor of the system 300 or semiautomatically) the emission of microwave energy radiation and thermal energy respectively from the therapy device 104.

A pump system 341 is shown coupled to the coolant source 340. The pump system 341 is coupled to a fluid reservoir system. A variety of cryogens may be stored in the fluid reservoir system. Suitable thermal transfer fluids for use in the thermal transfer fluid arrangement shown in Figure 8 include cold saline or cold saline and ethanol mixture, Freon or other fluorocarbon refrigerants, nitrous oxide, liquid nitrogen, and liquid carbon dioxide, for example.

Various embodiments disclosed herein are generally described in the context of ablation of perivascular renal nerves for control of hypertension. It is understood, however, that embodiments of the disclosure have applicability in other contexts, such as performing ablation from within other vessels of the body, including other arteries, veins, and vasculature (e.g., cardiac and urinary vasculature and vessels), and other tissues of the body, including various organs (e.g., the prostate for BPH ablation).

It is to be understood that even though numerous characteristics of various embodiments have been set forth in the foregoing description, together with details of the structure and function of various embodiments, this detailed description is illustrative only, and changes may be made in detail, especially in matters of structure and arrangements of parts illustrated by the various embodiments to the full extent indicated by the broad general meaning of the terms in which the appended claims are expressed.

## Claims

1. An apparatus, comprising:
a catheter arrangement comprising a flexible shaft and configured to deployment into the body;
a cooling arrangement provided at a distal end of the shaft and configured to freeze non-targeted body tissue while neighboring target body tissue remains unfrozen;
a microwave emitter configured to propagate microwave energy through the frozen non-targeted tissue and to heat the unfrozen target tissue to a temperature of 55°C or higher; and a measuring device configured to measure a depth of ice formation within the non-targeted tissue.

2. The apparatus of claim 1, wherein the catheter arrangement comprises a balloon within which at least the cooling arrangement is situated.

3. The apparatus of claim 1, wherein the catheter arrangement comprises a balloon within which the cooling arrangement and the microwave emitter are situated.

4. The apparatus of claim 1, wherein the measuring device comprises an ultrasound device or an opto-acoustic device configured to measure a depth of ice formation within the non-targeted tissue.

5. The apparatus of claim 1, wherein the cooling arrangement:
is configured to receive a thermal transfer fluid via the lumen arrangement; or
comprises a phase-change cryothermal apparatus configured to receive a cryogen liquid and output spent gas resulting from the cryothermal phase-change; or
comprises a heat exchange apparatus configured to receive a liquid coolant capable of causing freezing of the renal artery wall; or
comprises a solid-state thermoelectric cooling device.

6. The apparatus of claim 1, comprising one or more temperature sensors configured to sense a temperature at or proximate the non-targeted tissue.

7. The apparatus of claim 1, wherein the microwave emitter and the cooling arrangement are provided on a common catheter.

8. The apparatus of claim 1, wherein the microwave emitter and the cooling arrangement are provided on individual catheters, respectively.

9. The apparatus of claim 1, wherein the lumen arrangement comprises a guide lumen dimensioned to receive a guidewire.

10. The apparatus of claim 1, wherein the cooling arrangement is further configured to provide cooling to the microwave emitter.

11. The apparatus according to any of the preceding claims, wherein:
the shaft has a length sufficient to access a patient's renal artery relative to a percutaneous access location;
the cooling arrangement is dimensioned for deployment within the renal artery, the cooling arrangement configured to freeze tissue of a wall of the renal artery while target tissue adjacent the renal artery wall including perivascular renal nerve tissue remains unfrozen; and
the microwave emitter is configured to propagate microwave energy through the frozen renal artery wall to heat the unfrozen target tissue to a temperature sufficient to ablate perivascular renal nerve tissue included within the target tissue with no or negligible thermal damage to at least an inner portion of the renal artery wall.

12. The apparatus according to any of claim 1 through claim 10, wherein:
the shaft has a length sufficient to access a patient's renal artery relative to a percutaneous access location;
the cooling arrangement comprises a cryoballoon dimensioned for deployment within the renal artery, the cryoballoon configured to freeze tissue of a wall of the renal artery while target tissue adjacent the renal artery wall including perivascular renal nerve tissue remains unfrozen; and
the microwave emitter is configured to propagate microwave energy through the frozen renal artery wall to heat the unfrozen target tissue to a temperature sufficient to ablate perivascular renal nerve tissue included within the target tissue with no or negligible thermal damage to at least an inner portion of the renal artery wall.

13. The apparatus of claim 12, wherein the microwave emitter is encompassed at least in part by the cryoballoon.

14. The apparatus according to any of the preceding claims, comprising an external system coupled to the proximal end of the catheter arrangement, the system configured to control power delivered to the microwave emitter and coolant delivered to the cooling arrangement.

## Patentansprüche

1. Vorrichtung, die aufweist:
eine Katheteranordnung, die einen flexiblen Schaft aufweist und zum Einsatz im Körper konfiguriert ist;
eine Kühlanordnung, die an einem distalen Ende des Schafts vorgesehen und konfiguriert ist, nicht als Ziel definiertes Körpergewebe einzufrieren, während benachbartes Zielkörpergewebe nicht gefroren bleibt;
eine Mikrowellenemissionsquelle, die konfiguriert ist, Mikrowellenenergie durch das gefrorene, nicht als Ziel definierte Gewebe auszubreiten und das nicht gefrorene Zielgewebe auf eine Temperatur von 55°C oder mehr zu erwärmen; und
eine Messvorrichtung, die konfiguriert ist, eine Tiefe der Eisbildung innerhalb des nicht als Ziel definierten Gewebes zu messen.

2. Vorrichtung nach Anspruch 1, wobei die Katheteranordnung einen Ballon aufweist, in dem sich mindestens die Kühlanordnung befindet.

3. Vorrichtung nach Anspruch 1, wobei die Katheteranordnung einen Ballon aufweist, in dem sich die Kühlanordnung und die Mikrowellenemissionsquelle befinden.

4. Vorrichtung nach Anspruch 1, wobei die Messvorrichtung eine Ultraschallvorrichtung oder eine optoakustische Vorrichtung aufweist, die konfiguriert ist, eine Tiefe der Eisbildung innerhalb des nicht als Ziel gesetzten Gewebes zu messen.

5. Vorrichtung nach Anspruch 1, wobei die Kühlanordnung:
konfiguriert ist, ein Wärmeübertragungsfluid über die Lumenanordnung aufzunehmen; oder
eine Cryothermie-Phasenübergangsvorrichtung aufweist, die konfiguriert ist, eine Tieftemperaturflüssigkeit aufzunehmen und verbrauchtes Gas abzugeben, die aus dem Cryothermie-Phasenübergang herrührt; oder
eine Wärmetauschervorrichtung aufweist, die konfiguriert ist, ein flüssiges Kühlmittel aufzunehmen, das imstande ist, ein Einfrieren der Nierenarterienwand zu bewirken; oder
eine thermoelektrische Festkörperkühlvorrichtung aufweist.

6. Vorrichtung nach Anspruch 1, die einen oder mehrere Temperatursensoren aufweist, die konfiguriert sind, eine Temperatur an oder nahe dem nicht als Ziel definierten Gewebe abzutasten.

7. Vorrichtung nach Anspruch 1, wobei die Mikrowellenemissionsquelle und die Kühlanordnung an einem gemeinsamen Katheter vorgesehen sind.

8. Vorrichtung nach Anspruch 1, wobei die Mikrowellenemissionsquelle bzw. die Kühlanordnung an einzelnen Kathetern vorgesehen sind.

9. Vorrichtung nach Anspruch 1, wobei die Lumenanordnung ein Führungslumen aufweist, das bemessen ist, einen Führungsdraht aufzunehmen.

10. Vorrichtung nach Anspruch 1, wobei die Kühlanordnung ferner konfiguriert ist, für die Mikrowellenemissionsquelle eine Kühlung bereitzustellen.

11. Vorrichtung nach einem der vorhergehenden Ansprüche, wobei:
der Schaft eine Länge aufweist, die ausreicht, um relativ zu einer perkutanen Zugangsstelle Zugang zur Nierenarterie zu erlangen;
die Kühlanordnung zum Einsatz innerhalb der Nierenarterie bemessen ist, die Kühlanordnung konfiguriert ist, Gewebe einer Wand der Nierenarterie einzufrieren, während Zielgewebe benachbart zur Nierenarterienwand einschließlich des perivaskulären Nierennervengewebes nicht gefroren bleibt; und
die Mikrowellenemissionsquelle konfiguriert ist, Mikrowellenenergie durch die gefrorene Nierenarterienwand auszubreiten, um das nicht gefrorene Zielgewebe auf eine Temperatur zu erwärmen, die ausreicht, das perivaskuläre Nierennervengewebe abzutragen, das im Zielgewebe enthalten ist, ohne eine oder mit einer vernachlässigbaren thermischen Schädigung an mindestens einem inneren Abschnitt der Nierenarterienwand.

12. Vorrichtung nach einem der Ansprüche 1 bis 10, wobei:
der Schaft eine Länge aufweist, die ausreicht, um relativ zu einer perkutanen Zugangsstelle Zugang zur Nierenarterie zu erlangen;
die Kühlanordnung einen Kryoballon aufweist, der zum Einsatz in der Nierenarterie bemessen ist, wobei der Kryoballon konfiguriert ist, Gewebe einer Wand der Nierenarterie einzufrieren, während Zielgewebe benachbart zur Nierenarterienwand einschließlich des perivaskulären Nierennervengewebes nicht gefroren bleibt; und
die Mikrowellenemissionsquelle konfiguriert ist, Mikrowellenenergie durch die gefrorene Nierenarterienwand auszubreiten, um das nicht gefrorene Zielgewebe auf eine Temperatur zu erwärmen, die ausreicht, das perivaskuläre Nierennervengewebe abzutragen, das im Zielgewebe enthalten ist, ohne eine oder mit einer vernachlässigbaren thermischen Schädigung an mindestens einem inneren Abschnitt der Nierenarterienwand.

13. Vorrichtung nach Anspruch 12, wobei die Mikrowellenemissionsquelle mindestens teilweise durch den Kryoballon umgeben ist.

14. Vorrichtung nach einem der vorhergehenden Ansprüche, die ein externes System aufweist, das mit dem proximalen Ende der Katheteranordnung gekoppelt ist, wobei das System konfiguriert ist, die an die Mikrowellenemissionsquelle abgegebene Leistung und das der Kühlanordnung zugeführte Kühlmittel zu steuern.

## Revendications

1. Appareil, comprenant :
une unité de cathéter comprenant une tige flexible et prévue pour être déployée dans le corps ;
une unité de refroidissement prévue à une extrémité distale de la tige et prévue pour congeler un tissu corporel non ciblé alors que le tissu corporel ciblé environnant reste non congelé ;
un émetteur de micro-ondes prévu pour diffuser une énergie micro-ondes au travers du tissu corporel non ciblé congelé et pour chauffer le tissu corporel ciblé non congelé à une température égale ou supérieure à 55° C ; et
un dispositif de mesure prévu pour mesurer une profondeur de formation de glace à l'intérieur du tissu non ciblé.

2. Appareil selon la revendication 1, où l'unité de cathéter comporte un ballonnet à l'intérieur duquel au moins l'unité de refroidissement est disposée.

3. Appareil selon la revendication 1, où l'unité de cathéter comporte un ballonnet à l'intérieur duquel l'unité de refroidissement et l'émetteur de micro-ondes sont disposés.

4. Appareil selon la revendication 1, où le dispositif de mesure comporte un dispositif à ultrasons ou un dispositif opto-acoustique prévu pour mesurer une profondeur de formation de glace à l'intérieur du tissu non ciblé.

5. Appareil selon la revendication 1, où l'unité de refroidissement :
est prévue pour recevoir un fluide de transfert thermique par l'unité de lumière ; ou
comporte un appareil cryothermique à changement de phase prévu pour recevoir un liquide cryogénique et évacuer des gaz usés suite au changement de phase cryothermique ; ou
comporte un appareil d'échange de chaleur prévu pour recevoir un réfrigérant liquide apte à congeler la paroi d'artère rénale ; or
comporte un dispositif de refroidissement thermoélectrique à semi-conducteur.

6. Appareil selon la revendication 1, comprenant un ou plusieurs capteurs de température prévus pour détecter une température sur le tissu non ciblé ou à proximité de celui-ci.

7. Appareil selon la revendication 1, où l'émetteur de micro-ondes et l'unité de refroidissement sont prévus sur un cathéter commun.

8. Appareil selon la revendication 1, où l'émetteur de micro-ondes et l'unité de refroidissement sont prévus sur un cathéter individuel respectif.

9. Appareil selon la revendication 1, où l'unité de lumière comporte une lumière de fil-guide dimensionnée pour recevoir un fil-guide.

10. Appareil selon la revendication 1, où l'unité de refroidissement est en outre prévue pour refroidir l'émetteur de micro-ondes.

11. Appareil selon l'une des revendications précédentes, où :
la tige a une longueur suffisante pour accéder à une artère rénale d'un patient par rapport à un site d'accès percutané ;
l'unité de refroidissement est dimensionnée pour un déploiement à l'intérieur de l'artère rénale, l'unité de refroidissement étant prévue pour congeler le tissu d'une paroi de l'artère rénale alors que le tissu ciblé adjacent à la paroi d'artère rénale comprenant le tissu nerveux rénal péri-vasculaire reste non congelé ; et
l'émetteur de micro-ondes est prévu pour diffuser une énergie micro-ondes au travers de la paroi d'artère rénale congelée pour chauffer le tissu ciblé non congelé à une température suffisante pour l'ablation du tissu nerveux rénal péri-vasculaire compris à l'intérieur du tissu ciblé, sans lésion thermique, ou avec une lésion thermique négligeable sur au moins une partie intérieure de la paroi d'artère rénale.

12. Appareil selon l'une des revendications 1 à 10, où :
la tige a une longueur suffisante pour accéder à une artère rénale d'un patient par rapport à un site d'accès percutané ;
l'unité de refroidissement comporte un cryoballon dimensionné pour un déploiement à l'intérieur de l'artère rénale, le cryoballon étant prévu pour congeler le tissu d'une paroi de l'artère rénale alors que le tissu ciblé adjacent à la paroi d'artère rénale comprenant le tissu nerveux rénal péri-vasculaire reste non congelé ; et
l'émetteur de micro-ondes est prévu pour diffuser une énergie micro-ondes au travers de la paroi d'artère rénale congelée pour chauffer le tissu ciblé non congelé à une température suffisante pour l'ablation du tissu nerveux rénal péri-vasculaire compris à l'intérieur du tissu ciblé, sans lésion thermique, ou avec une lésion thermique négligeable sur au moins une partie intérieure de la paroi d'artère rénale.

13. Appareil selon la revendication 12, où l'émetteur de micro-ondes est entouré au moins en partie par le cryoballon.

14. Appareil selon l'une des revendications précédentes, comprenant un système externe relié à l'extrémité proximale de l'unité de cathéter, ledit système étant prévu pour commander le courant fournie à l'émetteur de micro-ondes et le réfrigérant fourni à l'unité de refroidissement.
